# EUROPÄISCHE PATENTSCHRIFT

(11) **EP 0 590 425 B1**
(45) Veröffentlichungstag und Bekanntmachung des Hinweises auf die Patenterteilung: **24.01.2001**
(21) Anmeldenummer: 93114919.9
(22) Anmeldetag: 16.09.1993
(51) Int. Cl.: A01N 61/00, A01N 51/00, A01N 47/44, A01N 47/40, A01N 43/86, A01N 43/50, A01N 43/40

(54) **Bekämpfung von parasitierenden Krebsen an Fischen**
Control of crustaceous fish parasites
Lutte contre les crustacés parasites des poissons

(30) Priorität: 29.09.1992 DE 4232561
(43) Veröffentlichungstag der Anmeldung: 06.04.1994
(73) Patentinhaber: BAYER AG, 51368 Leverkusen (DE)
(72) Erfinder: Löhr, Reinhold, Dr., D-51469 Bergisch Gladbach (DE); Mundt, Hans-Christian, Dr., D-40699 Erkrath (DE); Andrews, Peter, Dr., D-42115 Wuppertal (DE)

(56) Entgegenhaltungen:
- EP-A- 0 163 855
- EP-A- 0 292 822
- EP-A- 0 407 343
- CENTRAL PATENTS INDEX, BASIC ABSTRACTS JOURNAL Week 7718, 1977 Derwent Publications Ltd., London, GB; AN 77-31877Y/18 SANKYO K.K. & JP-A-5 203 812
- CENTRAL PATENTS INDEX, BASIC ABSTRACTS JOURNAL Week 7542, Derwent Publications Ltd., London, GB; AN 75-69434W/42 & JP-A-49 102 823 (KUMIAI CHEM. IND. K.K.)
- BIOLOGICAL ABSTRACTS, Bd. 90, Nr. 2, 15 Juli 1990, Philadelphia, PA, US; Zusammenfassung Nr. 18549, D.B.SATELLE ET AL.: 'Actions of the insecticide 2-(nitromethylene)- tetrahydro-1,3-thiazine on insect and vertebrate nictonic acetylcholine receptors'
- BIOLOGICAL ABSTRACTS, Bd. 95, Nr. 1, 01 Januar 1993, Philadelphia, PA, US; Zusammenfassung Nr. 5222, C.J. CHURCH ET AL.: 'Properties of alpha-bungarotoxin binding cholinergic receptors in pyrethroid susceptible and resistant tobacco budworm moths'
- DATABASE WPI Week 9031, 1990 Derwent Publications Ltd., London, GB; AN 90-231525/31 & CA-A-2 003 459 (SALK INST FOR BIOL STUD)

## Beschreibung

Die vorliegende Erfindung betrifft die Bekämpfung von fischparasitierenden Krebsen durch Mittel die Agonisten oder Antagonisten der nicotinergen Acetylcholinrezeptoren von Insekten enthalten.

Agonisten oder Antagonisten der nicotinergen Acetylcholinrezeptoren von Insekten sind bekannt aus den folgenden Publikationen:
Europäische Offenlegungsschriften Nr. 464 830, 428 941, 425 978, 386 565, 383 091, 375 907, 364 844, 315 826, 259 738, 254 859, 235 725, 212 600, 192 060, 163 855, 154 178, 136 636, 303 570, 302 833, 306 696, 189 972, 455 000, 135 956, 471 372, 302 389;
Deutsche Offenlegungsschriften Nr. 3 639 877, 3 712 307;
Japanische Offenlegungsschriften Nr. 03 220 176, 02 207 083, 63 307 857, 63 287 764, 03 246 283, 04 9371, 03 279 359, 03 255 072;
US-Patentschriften Nr. 5 034 524, 4 948 798, 4 918 086, 5 039 686. 5 034 404;
PCT-Anmeldungen Nr. WO 91/17 659, 91/4965;
Französische Anmeldungen Nr. 2611 114;
Brasilianische Anmeldung Nr. 88 03 621.

Diese Verbindungen werden z.T. unter dem Begriff Nitromethylene und damit verwandte Verbindungen zusammengefaßt.

Die intensive Fischzucht erleidet erhebliche wirtschaftliche Verluste durch Schädigungen der Fische, die durch fischparasitierende Krebse wie z.B. die Lachs- oder Seelaus hervorgerufen werden. Behandlungen gegen diese Parasiten mit Metrifonat oder Dichlorvos sind bekannt. Diese Wirkstoffe müssen in verhältnismäßig hohen Konzentrationen eingesetzt werden und erfordern eine lange Behandlungsdauer.

Andere Verbindungen zur Bekämpfung von Fischparasiten sind aus EP-OS 407 343 bekannt.

Es wurde nun gefunden, daß man Agonisten oder Antagonisten der nicotinergen Acetylcholinrezeptoren von Insekten entsprechend der Formel (I) hervorragend zur Bekämpfung parasitierender Krebse bei Fischen einsetzen kann;

### Verbindungen der Formel (I)

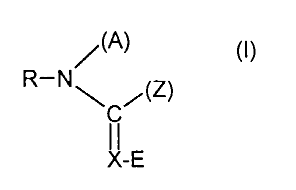

in welcher
- R: für Wasserstoff, gegebenenfalls substituierte Reste Acyl, Alkyl, Aryl, Aralkyl, Heteroaryl oder Heteroarylalkyl steht;
- A: für eine monofunktionelle Gruppe aus der Reihe Wasserstoff und gegebenenfalls substituierte Reste, Acyl, Alkyl oder Aryl steht oder für eine bifunktionelle Gruppe steht, die mit dem Rest Z verknüpft ist;
- E: für einen elektronenziehenden Rest steht;
- X: für die Reste -CH= oder -N= steht, wobei der Rest -CH= anstelle eines H-Atoms mit dem Rest Z verknüpft sein kann;
- Z: für eine monofunktionelle Gruppe aus der Reihe Alkyl, -O-R, -S-R, -NR-R steht oder für eine bifunktionelle Gruppe steht, die mit dem Rest A oder dem Rest X verknüpft ist.

Als bevorzugte Reste in Verbindungen der Formel (I) seien genannt:
für R und gegebenenfalls A und Z:

Als Acylreste: Formyl, Alkylcarbonyl, Arylcarbonyl, Alkylsulfonyl, Arylsulfonyl, (Alkyl-)-(Aryl-)-phosphoryl, die ihrerseits substituiert sein können.

Als Alkyl: C₁₋₁₀-Alkyl, insbesondere C₁₋₄-Alkyl, im einzelnen Methyl, Ethyl, i-Propyl, sec.- oder t.-Butyl, die ihrerseits substituiert sein können.

Als Aryl: Phenyl, Naphthyl, insbesondere Phenyl.

Als Aralkyl: Phenylmethyl, Phenethyl.

Als Heteroaryl: Heteroaryl mit bis zu 10 Ringatomen und N, O, S insbesondere N als Heteroatomen. Im einzelnen seien genannt Thiophenyl, Furyl, Thiazolyl, Imidazolyl, Pyridyl, Benzthiazolyl.

Als Heteroarylalkyl: Heteroarylmethyl, Heteroarylethyl mit bis zu 6 Ringatomen und N, O, S, insbesondere N als Heteroatomen.
für A und gegebenenfalls Z ferner:
als bifunktionelle Gruppe: gegebenenfalls substituiertes Alkylen mit 1-4, insbesondere 1-2 C-Atomen, oder andere bifunktionelle Gruppen, die A und Z erlauben, gemeinsam mit den Atomen, an welche sie gebunden sind, einen gesättigten oder ungesättigten heterocyclischen Ring zu bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei Alkyl der N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.

Als Beispiele für den heterocyclischen Ring seien Pyrrolidin, Piperidin, Piperazin, Hexamethylenimin, Morpholin und N-Methylpiperazin genannt.
für E: insbesondere NO₂, CN, Halogenalkylcarbonyl wie 1,5-Halogen-C₁₋₄-carbonyl, insbesondere COCF₃;
für Z außerdem: als mit dem Rest X verknüpfte bifunktionelle Gruppe: Gruppen, die es Z erlauben, gemeinsam mit dem Atom, an welches es gebunden ist und dem Rest 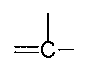 an Stelle von X einen gesättigten oder ungesättigten heterocyclischen Ring zu bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei die Alkyl oder N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.
Als Beispiele für den heterocyclischen Ring seien Pyrrolidin, Piperidin, Piperazin, Hexamethylenimin, Morpholin und N-Methylpiperazin genannt.

Als Substituenten seien beispielhaft und vorzugsweise aufgeführt:

Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome vorzugsweise für Fluor, Chlor oder Brom, insbesondere Fluor stehen, wie Trifluormethyl; Hydroxy; Halogen, vorzugsweise Fluor, Chlor, Brom und Iod, insbesondere Fluor, Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n-butylamino; Carboxyl; Carbalkoxy mit vorzugsweise 2 bis 4, insbesondere 2 oder 3 Kohlenstoffatomen, wie Carbomethoxy und Carboethoxy; Sulfo (-SO₃H); Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl sowie Heteroarylamino und Heteroalkylamino wie Chlorpyridylamino und Chlorpyridylmethylamino.

Als ganz besonders bevorzugt erfindungsgemäß verwendbare Verbindungen seien Verbindungen der allgemeinen Formeln II und III genannt: 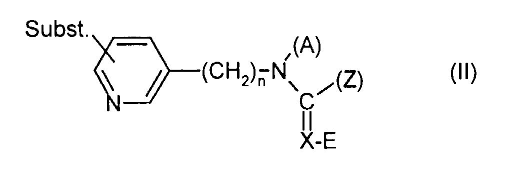 in welcher
- n: für 1 oder 2 steht,

Subst. für einen der oben aufgeführten Substituenten, besonders für Halogen, insbesondere für Chlor steht,
A, Z, X und E die oben angegebenen Bedeutungen haben, 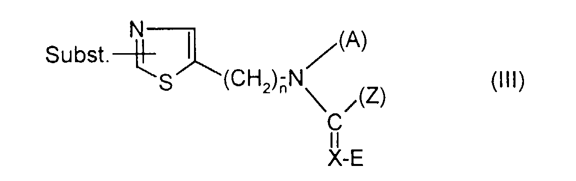 in welcher
die Reste die oben genannte Bedeutung haben.

Im einzelnen seien folgende Verbindungen genannt:

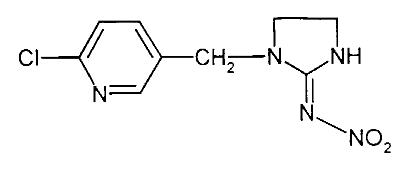

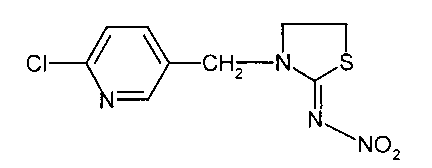

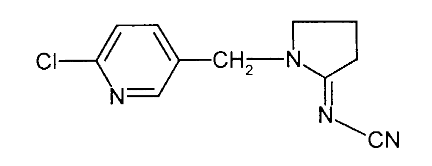

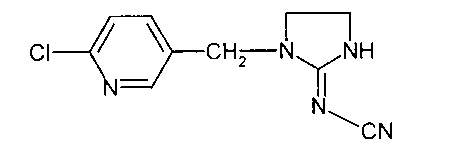

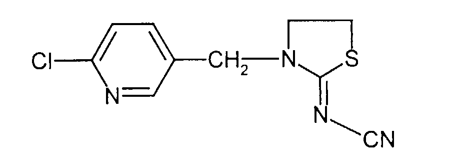

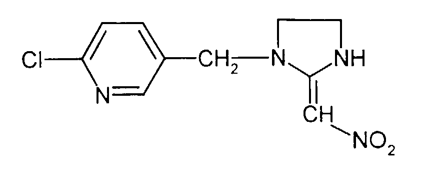

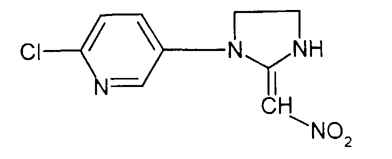

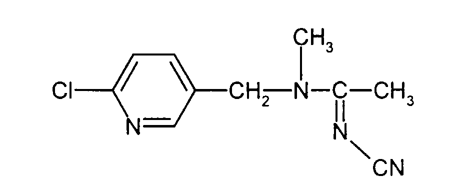

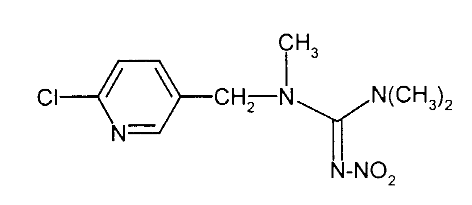

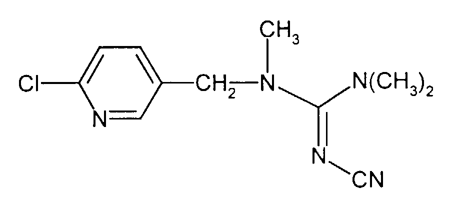

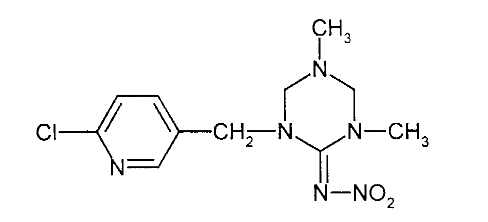

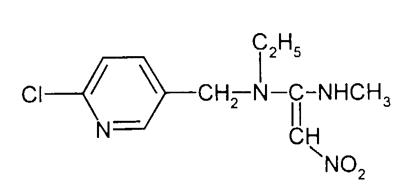

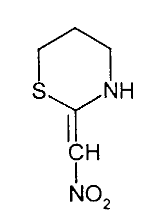

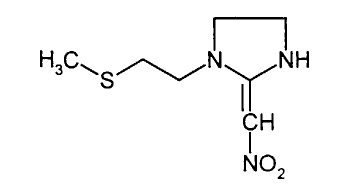

Wie bereits erwähnt, lassen sich die erfindungsgemäß verwendbaren Verbindungen hervorragend zur Bekämpfung von fischparasitierenden Krebsen einsetzen. Dazu zählen die Copepodae (Hüpferlinge) mit den Gattungen
Ergasilus
Bromolochus
Chondracaushus
Caligus (→ Caligus curtus)
Lepeophtheirus (→ L. salmonis)
Elythrophora
Dichelestinum
Lamproglenz
Hatschekia
Legosphilus
Symphodus
Ceudrolasus
Pseudocycmus
Lernaea
Lernaeacera
Pennella
Achthares
Basanistes
Salmincola
Brachiella
Epibrachiella
Pseudotracheliastes
und den Familien
Ergasilidae
Bromolochiadae
Chondracanthidae
Caligidae
Dichelestiidae
Philichthyidae
Pseudocycnidae
Lernaeidae
Lernaeipodidae
Sphyriidae
Cecropidae
sowie die Branchiuriae (Kiemenschwänze) mit den Familien Argulidae und den Gattungen Argulus spec.;
sowie die Cirripediae (Rauhenfüßer) und Ceratothoa gandichaugii.

Zu den Fischen gehören Nutz-, Zucht-, Aquarien- und Zierfische aller Altersstufen, die in Süß-, Salz- und Brackwasser leben. Zu den Nutz- und Zuchtfischen zählen z.B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabream (Pagurus major), Seabass (Dicentrarchus labrax), Grey mullet (Mugilus caphalus), Pompano. Gilthread seabream (Sparus auratus), Tilapia spp., Chichliden-Arten wie z.B. Plagioscion, Channel catfish. Besonders geeignet sind die erfindungsgemäßen Mittel zur Behandlung von Fischbrut z.B. Karpfen von 2 bis 4 cm Körperlänge. Sehr gut geeignet sind die Mittel auch in der Aalmast.

Die Behandlung der Fische erfolgt entweder oral z.B. über das Futter oder durch Badbehandlung z.B. "medizinisches Bad", in das die Fische eingesetzt und in dem sie eine zeitlang (Minuten bis mehrere Stunden) z.B. beim Umsetzen von einem Zuchtbecken zum anderen gehalten werden. In besonderen Fällen kann die Behandlung auch parenteral z.B. durch Injektion erfolgen.

Es kann auch eine vorübergehende oder dauernde Behandlung des Lebensraums der Fische z.B. in Netzkäfigen, ganzer Teichanlagen, Aquarien, Tanks oder Becken in denen die Fische gehalten werden, erfolgen.

Der Wirkstoff wird in Zubereitungen verabreicht, die den Anwendungen angepaßt sind.

Zubereitungen zu oralen Anwendungen sind Pulver, Granulate, Lösungen, Emulsions- oder Suspensionskonzentrate, die als Futterzusätze mit dem Futter homogen vermischt werden.

Zubereitungen zur Anwendung als Bad oder zur Behandlung des Lebensraumes sind Pulver, Granulate, Lösungen, Emulsions- oder Suspensionskonzentrate, Emulsionen oder Suspensionen, Tabletten oder der Wirkstoff selbst. Die Formulierungen können in verdünnter oder unverdünnter Form vom Anwender zur Anwendung gebracht werden.

Die Zubereitungen werden in an sich bekannter Weise hergestellt, indem man den Wirkstoff mit festen oder flüssigen Trägerstoffen gegebenenfalls unter Zusatz weiterer Hilfsstoffe wie Emulgier- oder Dispergiermittel, Lösungsvermittler, Farbstoffe, Antioxidantien, Konservierungsstoffe vermischt, granuliert, mahlt und/oder kompaktiert.

Im Vergleich zu den gebräuchlichen phosphororganischen Verbindungen sind Nitromethylene im allgemeinen für die Anwendungskonzentration ausreichend wasserlöslich und können daher auch unverdünnt eingesetzt werden.

Besser handhabbar sind jedoch Präparate, in denen der Wirkstoff in verdünnter Form vorliegt. Als Verdünnungsmittel kommen für Fische und andere Meerestiere und -pflanzen untoxische Substanzen, die flüssig oder auch fest sein können und unmittelbar vor der erfindungsgemäßen Verwendung auch Wasser in Frage.

Für den praktischen Einsatz sind auch Folien geeignet, die den Wirkstoff in einer leicht wasserlöslichen Matrix enthalten, oder Folien, aus denen der Wirkstoff über die Zeit der Anwendung herausdiffundiert.

Der Wirkstoff selbst, dessen gemahlene Form oder dessen Feststoffformulierungen können in wasserlöslichen Verpackungen z.B. in Polyvinylalkohol-Beuteln mitsamt der geschlossenen Packung zur Anwendung kommen. Der Anwender ist nicht mehr dem Wirkstoff oder dessen Formulierungen ausgesetzt.

Auch halbfeste Anwendungsformen können zur Badbehandlung eingesetzt werden. Aus öligen oder aus Fettmatrizes wird der darin suspendierte oder gelöste Wirkstoff ausgewaschen. Eine Steuerung der Freisetzung ist durch Auswahl der Hilfsstoffe, Konzentration des Wirkstoffes und Form (Oberfläche) möglich; Komprimate oder Schmelzen von Hartfetten, in denen der Wirkstoff vorliegt, sind zur Anwendung ebenfalls geeignet.

Die erfindungsgemäßen verdünnten Mittel werden hergestellt, indem der Wirkstoff der Formel (I) mit flüssigen und/oder festen Formulierungshilfsstoffen durch schrittweises Vermischen und/oder Vermahlen derart in Kontakt gebracht werden, daß eine anwendungskonforme optimale Entfaltung der antiparasitären Aktivität der Formulierung erzielt wird.

Die Formulierungsschritte können durch Kneten, Granulieren (Granulate) und gegebenenfalls Pressen, Extrudieren oder Spritzgießen (Pellets, Tabletten) ergänzt werden.

Als Formulierungshilfsstoffe dienen beispielsweise für die Meeresflora und -fauna untoxische feste Trägerstoffe, Lösungsmittel und gegebenenfalls oberflächenaktive Stoffe (Tenside).

Zur Bereitung der erfindungsgemäßen Mittel werden folgende Formulierungs hilfsstoffe verwendet:

Feste Trägerstoffe wie z.B. Kaolin, Talkum, Bentonit, Kochsalz, Calciumphosphat, Kohlenhydrate, Cellulosepulver, Baumwollsaatmehl, Polyethylenglykohlether, gegebenenfalls Bindemittel wie z.B. Gelatine, lösliche Cellulosederivate, falls notwendig unter Zusatz von oberflächenaktiven Stoffen wie ionischen oder nichtionischen Dispersionsmittteln; ferner natürliche Gesteinsmehle wie Calcit, Montmorrillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinertes Pflanzenmaterial verwendet werden. Auch sorptive organische Materialien z.B. Polyacrylate können mit dem Wirkstoff versetzt und zur Anwendung gebracht werden.

Als Lösungsmittel kommen in Frage: Aromatische Kohlenwasserstoffe, bevorzugt die Fraktionen C₈-C₁₂, wie z.B. Xylolgemische oder substituierte Naphthaline, Phthalsäureester wie Dibutyl- oder Dioctylphthalat; aliphatische Kohlenwasserstoffe wie z.B. Cyclohexan oder Paraffine, Alkohole und Glykole sowie deren Ether und Ester, wie z.B. Ethanol, Ethylenglykol, Ethylenglykolmonomethyl- oder Ethylether, Ketone wie z.B. Cyclohexanon, stark polare Lösungsmittel wie z.B. N-Methyl-2-pyrrolidon, Dimethylsulfoxid oder Dimethylformamid, sowie gegebenenfalls epoxydierte Pflanzenöle wie z.B. epoxydiertes Kokosnussöl oder Sojaöl und Wasser.

Als oberflächenaktive Verbindungen kommen je nach Art des zu formulierenden Wirkstoffes der Formel (I) nicht-ionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren (C₁₀-C₂₂), wie z.B. die Na- oder K-Salze der Öl- oder Stearinsäure, oder von natürlichen Fettsäuregemischen, die z.B. aus Kokosnuss- oder Talgöl gewonnen werden können, genannt.

Häufig werden sogenannte synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalinsulphonsäure oder eines Naphthalinsulfonsäure-Formaldehydkondensationsproduktes.

Ferner können auch entsprechende Phosphate, wie z.B. Salze des Phosphorsäureesters eines p-Nonylphenol-(4-14)-Ethylenoxyd-Adduktes oder Phospholipide, als Formulierungshilfsstoffe Anwendung finden.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nicht-ionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht-ionischer Tenside seien Nonylphenolpolyethoxyethanole, Ricinusölpolyglykolether, Polypropylen-Polyethylenoxyaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitantrioleat in Betracht.

Bei den kationischen Tensiden handelt es sich um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"McCutcheon's Detergents and Emulsifiers Annual" MC Publishing Corp., New Jersey, 1990; Helmut Stache "Tensid-Taschenbuch" Carl Hanser-Verlag München/Wien 1981.

Als Bindemittel für wasserlösliche Granulate oder Tabletten kommen chemisch abgewandelte, in Wasser oder Alkohol lösliche, polymere Naturstoffe in Frage, wie Stärke-, Cellulose- oder Proteinderivate (z.B. Methylcellulose, Carboxymethylcellulose, Ethylhydroxyethylcellulose, Proteine wie Zein, Gelatine und dergleichen) sowie synthetische Polymere wie z.B. Polyvinylalkohol, Polyvinylpyrrolidon etc. Ferner sind in Tabletten Füllstoffe, (z.B. Stärke, mikrokristalline Cellulose, Zucker, Milchzucker etc.), Gleitmittel und Sprengmittel enthalten.

Die Bad-Applikation der erfindungsgemäßen Mittel auf die zu bekämpfenden Parasiten kann so durchgeführt werden, daß die Mittel in Form von Lösungen, Emulsionen, Suspensionen, Pulvern oder Tabletten in den Käfig gegeben werden, wo sie durch die Bewegung der Fische und das durchströmende Wasser rasch aufgelöst und verteilt werden. Konzentrierte Lösungen können auch vor der Zugabe in die Käfige mit größeren Volumina von Wasser verdünnt werden. Konzentrationsprobleme in den Käfigen treten i.a. nicht auf, da die Fische bei jedem Öffnen der Käfige in Futtererwartung wild durcheinander wirbeln und für rasche Verdünnung sorgen.

Die erfindungsgemäßen antiparasitären Mittel enthalten in der Regel 0,1 bis 99 Gew.-%, insbesondere 0,1 bis 95 Gew.-% Wirkstoff der Formel (I), und 99,9 bis 1 Gew.-%, insbesondere 99,9 bis 5 Gew.-% eines festen oder flüssigen Zusatzstoffes, darunter 0 bis 25 Gew.-%, insbesondere 0,1 bis 25 Gew.-%, eines Tensides.

Während als Handelware eher konzentrierte Mittel bevorzugt werden, verwendet der Endverbraucher in der Regel verdünnte Mittel, die er durch Verdünnen der Handelsware mit Wasser erhält.

Solche Mittel können noch weitere Zusätze wie Stabilisatoren, Entschäumer, Viskositätsregulatoren, Bindemittel, Haftmittel sowie andere Wirkstoffe zur Erzielung spezieller Effekte enthalten.

Die Konzentration des Wirkstoffes bei der Anwendung hängt ab von Art und Dauer der Behandlung, sowie Alter und Zustand der behandelten Fische. Sie beträgt z.B. bei Kurzzeitbehandlung 0,1 bis 100 mg Wirkstoff pro Liter Wasser, bevorzugt 0,5 bis 10 mg pro Liter, bei einer Behandlungsdauer von 0,3 bis 4 Stunden.

Bei Teichbehandlungen können 0,01 bis 50 mg Wirkstoff pro Liter Wasser verwendet werden.

Zubereitungen zur Anwendung als Futterzusatz sind z.B. wie folgt zusammengesetzt:

| | | |
|---|---|---|
| a) | Wirkstoff der Formel (I) | 1-10 Gewichtsteile |
| | Sojabohnen-Protein | 49-90 Gewichtsteile |
| | gemahlenes Kalkmehl | 0-50 Gewichtsteile |

| | | |
|---|---|---|
| b) | Wirkstoff der Formel (I) | 0,5-10 Gewichtsteile |
| | Benzylalkohol | 0,08-1,4 Gewichtsteile |
| | Hydroxypropylmethylcellulose | 0-3,5 Gewichtsteile |
| | Wasser | Rest ad 100 |

Zubereitungen für die Badapplikation sind z.B. folgende Lösungen, Emulsionskonzentrate oder Suspensionskonzentrate.

| | | |
|---|---|---|
| c) | Wirkstoff der Formel (I) | 5,0 % |
| | anionischer Emulgator | 10,0 % |
| | N-Methylpyrrolidon | 25,0 % |
| | Mineralöl | 60,0 % |

| | | |
|---|---|---|
| d) | Wirkstoff der Formel (I) | 25,0 % |
| | anionischer Emulgator | 8,0 % |
| | nichtionischer Emulgator | 2,0 % |
| | Dimethylsulfoxid | 35,0 % |
| | N-Methylpyrrolidon | 30,0 % |

| | | |
|---|---|---|
| e) | Wirkstoff der Formel (I) | 30,0 % |
| | Harnstoff | 10,0 % |
| | Polyvinylalkohol | 0,5 % |
| | Gum (z.B. Xanthan Gum) | 0,4 % |
| | Konservierungsstoff | 0,1 % |
| | Wasser | 49,0 % |

### Beispiele

### Beispiel A

### In-vitro Test gegen Lachslaus

Glasschalen werden mit 40 ml Seewasser gefüllt und mit soviel 0,1 (Gewicht) oder 10 % (Gewicht) einer Lösung des Wirkstoffs in Ethanol versetzt, daß die gewünschte Anwendungskonzentration erreicht wird. Als Kontrolle dient wirkstofffreies Seewasser und Seewasser, das mit der entsprechenden Ethanolmenge ohne Wirkstoff versetzt ist.

In jede Glasschale werden 5 adulte Lachsläuse, die von natürlich infizierten Lachsen entfernt worden sind, gesetzt. Die Glasschalen werden bei 10 bis 12°C gehalten und nach 1, 2, 5 und 24 Stunden kontrolliert. Es wird angegeben, wieviel Testtiere leben bzw. wieviel Testtiere tot sind. Die Ergebnisse sind in Tabelle zusammengefaßt:

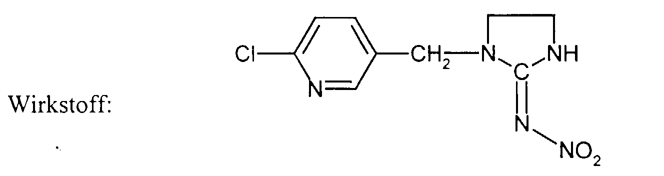

| | Zustand der Lachsläuse nach Stunden | | | |
|---|---|---|---|---|
| | **1** | **2** | **5** | **24** |
| Seewasser | 5 leben | | | 5 leben |
| Seewasser + 0,04 ml Ethanol | 5 leben | | | 5 leben |
| 1 ppm Wirkstoff | 4 leben | 5 tot | | |
| | 1 tot | | | |
| 100 ppm Wirkstoff | 5 tot | | | |

## Patentansprüche

1. Verwendung von Verbindungen der allgemeinen Formel I 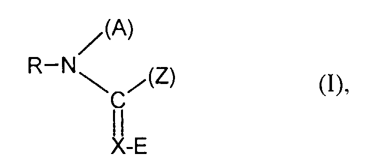 in welcher
R für Wasserstoff, gegebenenfalls substituierte Reste Acyl, Alkyl, Aryl, Aralkyl, Heteroaryl oder Heteroarylalkyl steht;
A für eine monofunktionelle Gruppe aus der Reihe Wasserstoff und gegebenenfalls substituierte Reste, Acyl, Alkyl oder Aryl steht oder für eine bifunktionelle Gruppe steht, die mit dem Rest Z verknüpft ist;
E für einen elektronenziehenden Rest steht;
X für die Reste -CH= oder -N= steht, wobei der Rest -CH= anstelle eines H-Atoms mit dem Rest Z verknüpft sein kann;
Z für eine monofunktionelle Gruppe aus der Reihe Alkyl, -O-R, -S-R, -NR-R steht oder für eine bifunktionelle Gruppe steht, die mit dem Rest A oder dem Rest X verknüpft ist.
zur Herstellung von Mitteln zur Bekämpfung von fischparasitierenden Krebsen bei Fischen.

2. Verwendung von Verbindungen der allgemeinen Formel (I) gemäß Anspruch 1, in welcher
R für Wasserstoff, gegebenenfalls substituiertes Formyl, Alkylcarbonyl, Arylcarbonyl, Alkylsulfonyl, Arylsulfonyl, (Alkyl-)-(Aryl-)-phosphoryl, C₁₋₁₀-Alkyl, Phenyl, Naphthyl, Phenylmethyl, Phenethyl, Heteroaryl mit bis zu 10 Ringatomen und N, O, S insbesondere N als Heteroatomen oder Heteroarylmethyl, Heteroarylethyl mit bis zu 6 Ringatomen und N, O, S, insbesondere N als Heteroatomen steht;
A für eine monofunktionelle Gruppe aus der Reihe Wasserstoff und gegebenenfalls substituiertes Formyl, Alkylcarbonyl, Arylcarbonyl, Alkylsulfonyl, Arylsulfonyl, (Alkyl-)-(Aryl-)-phosphoryl, C₁₋₁₀-Alkyl, Phenyl, Naphthyl, steht oder für gegebenenfalls substituiertes Alkylen mit 1 bis 4 C-Atomen, oder andere bifunktionelle Gruppen, die A und Z erlauben, gemeinsam mit den Atomen, an welche sie gebunden sind, einen gesättigten oder ungesättigten heterocyclischen Ring zu bilden steht,
E für NO₂, CN, Halogenalkylcarbonyl wie 1,5-Halogen-C₁₋₄-carbonyl, steht;
X für die Reste -CH= oder -N= steht, wobei der Rest -CH= anstelle eines H-Atoms mit dem Rest Z verknüpft sein kann;
Z für Gruppen, die es Z erlauben, gemeinsam mit dem Atom, an welches es gebunden ist und dem Rest =C- an Stelle von X einen gesättigten oder ungesättigten heterocyclischen Ring zu bilden, steht,
zur Herstellung von Mitteln zur Bekämpfung von fischparasitierenden Krebsen bei Fischen.

3. Verwendung von Verbindungen der allgemeinen Formel (I) gemäß Anspruch 1, in welcher
R für Wasserstoff, gegebenenfalls substituiertes Formyl, Alkylcarbonyl, Arylcarbonyl, Alkylsulfonyl, Arylsulfonyl, (Alkyl-)-(Aryl-)-phosphoryl, Methyl, Ethyl, i-Propyl, sec.- oder t.-Butyl, Phenyl, Thiophenyl, Furyl, Thiazolyl, Imidazolyl, Pyridyl, Benzthiazolyl oder Heteroarylmethyl, Heteroarylethyl mit Thiophenyl, Furyl, Thiazolyl, Imidazolyl, Pyridyl als Heteroarylrest;
A für eine monofunktionelle Gruppe aus der Reihe Wasserstoff und gegebenenfalls substituiertes Formyl, Alkylcarbonyl, Arylcarbonyl, Alkylsulfonyl, Arylsulfonyl, (Alkyl-)-(Aryl-)-phosphoryl, Methyl, Ethyl, i-propyl, sec.- oder t.-Butyl, Phenyl steht oder für gegebenenfalls substituiertes Alkylen mit 1 bis 2 C-Atomen, oder andere bifunktionelle Gruppen, die A und Z erlauben, gemeinsam mit den Atomen, an welche sie gebunden sind, einen gesättigten 5- bis 7-gliedrigen heterocyclischen Ring zu bilden der weitere 1 oder 2 gleiche oder verschiedene Heteroatome und/oder Heterogruppen enthält, steht,
E für NO₂, CN oder COCF₃ steht;
X für die Reste -CH= oder -N= steht, wobei der Rest -CH= anstelle eines H-Atoms mit dem Rest Z verknüpft sein kann;
Z für Gruppen, die es Z erlauben, gemeinsam mit dem Atom, an welches es gebunden ist und dem Rest =C- an Stelle von X einen gesättigten oder ungesättigten 5- bis 7-gliedrigen heterocyclischen Ring zu bilden, der weitere 1 oder 2 gleiche oder verschiedene Heteroatome und/oder Heterogruppen enthält, steht,
zur Herstellung von Mitteln zur Bekämpfung von fischparasitierenden Krebsen bei Fischen.

4. Verwendung von Verbindungen der allgemeinen Formel (I) gemäß Anspruich 1, in welcher
R für Wasserstoff, gegebenenfalls substituiertes Formyl, Alkylcarbonyl, Arylcarbonyl, Alkylsulfonyl, Arylsulfonyl, (Alkyl-)-(Aryl-)-phosphoryl, Methyl, Ethyl, i-Propyl, sec.- oder t.-Butyl, Phenyl, Thiophenyl, Furyl, Thiazolyl, Imidazolyl, Pyridyl, Benzthiazolyl oder Heteroarylmethyl, Heteroarylethyl mit Thiophenyl, Furyl, Thiazolyl, Imidazolyl, Pyridyl als Heteroarylrest;
A für eine monofunktionelle Gruppe aus der Reihe Wasserstoff und gegebenenfalls substituiertes Formyl, Alkylcarbonyl, Arylcarbonyl, Alkylsulfonyl, Arylsulfonyl, (Alkyl-)-(Aryl-)-phosphoryl, Methyl, Ethyl, i-Propyl, sec.- oder t.-Butyl, Phenyl steht oder für gegebenenfalls substituiertes Alkylen mit 1 bis 2 C-Atomen, oder andere bifunktionelle Gruppen, die A und Z erlauben, gemeinsam mit den Atomen, an welche sie gebunden sind, einen gesättigten oder ungesättigten 5- bis 7-gliedrigen heterocyclischen Ring zu bilden, der weitere 1 oder 2 gleiche oder verschiedene Heteroatome und/oder Heterogruppen aus der Gruppe Sauerstoff, Schwefel oder Stickstoff und als Heterogruppen N-Alkyl enthält, steht,
E für NO₂, CN oder COCF₃ steht;
X für die Reste -CH= oder -N= steht, wobei der Rest -CH= anstelle eines H-Atoms mit dem Rest Z verknüpft sein kann;
Z für Gruppen, die es Z erlauben, gemeinsam mit dem Atom, an welches es gebunden ist und dem Rest =C- an Stelle von X einen gesättigten oder ungesättigten 5- bis 7-gliedrigen heterocyclischen Ring zu bilden, der weitere 1 oder 2 gleiche oder verschiedene Heteroatome und/oder Heterogruppen aus der Gruppe Sauerstoff, Schwefel oder Stickstoff und als Heterogruppe N-Alkyl enthält, steht,
zur Herstellung von Mitteln zur Bekämpfung von fischparasitierenden Krebsen bei Fischen.

5. Verwendung gemäß Anspruch 1 von Verbindungen der allgemeinen Formel II und III: 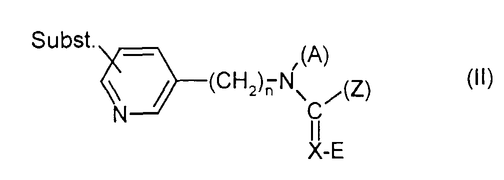 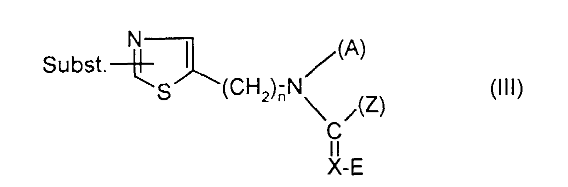 in welcher
n für 1 oder 2 steht,
Subst. für Halogen steht,
A, Z, X und E die in Anspruch 1 angegebenen Bedeutungen haben,
zur Herstellung von Mitteln zur Bekämpfung von fischparasitierenden Krebsen bei Fischen.

6. Verwendung gemäß Anspruch 1 von Imidacloprid der Formel 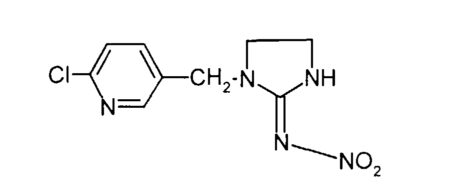 zur Herstellung von Mitteln zur Bekämpfung von fischparasitierenden Krebsen bei Fischen.

7. Verwendung von Verbindungen gemäß Ansprüchen 1-6 zur Herstellung von Mitteln zur Bekämpfung von fischparasitierenden Krebsen aus den Gattungen Caligus, Lepeophtheirus und Argulus.

8. Verwendung von Verbindungen gemäß Ansprüchen 1-6 zur Herstellung von Mitteln zur Bekämpfung von fischparasitierenden Krebsen bei Salmonidae.

## Claims

1. Use of compounds of the general formula I 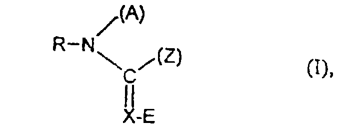 in which
R represents hydrogen or optionally substituted acyl, alkyl, aryl, aralkyl, heteroaryl or heteroarylalkyl radicals;
A represents a monofunctional group from the series consisting of hydrogen and optionally substituted acyl, alkyl or aryl radicals, or represents a bifunctional group which is linked to the Z radical;
E represents an electron-attracting radical;
X represents the -CH= or -N= radicals, with it being possible for the -CH= radical to be linked to the Z radical instead of an H atom;
Z represents a monofunctional group from the series consisting of alkyl, -O-R, -S-R and -NR-R, or represents a bifunctional group which is linked to the A radical or to the X radical,
for preparing agents for controlling fish-parasitizing lice in fish.

2. Use of compounds of the general formula (I) according to Claim 1, in which
R represents hydrogen or optionally substituted formyl, alkylcarbonyl, arylcarbonyl, alkylsulphonyl, arylsulphonyl, (alkyl-)-(aryl-)-phosphoryl, C₁₋₁₀-alkyl, phenyl, naphthyl, phenylmethyl, phenethyl, heteroaryl having up to 10 ring atoms and whose heteroatoms are N, O or S, in particular N, or heteroarylmethyl, heteroarylethyl having up to 6 ring atoms and whose heteroatoms are N, O or S, in particular N;
A represents a monofunctional group from the series consisting of hydrogen and optionally substituted formyl, alkylcarbonyl, arylcarbonyl, alkylsulphonyl, arylsulphonyl, (alkyl-)-(aryl-)-phosphoryl, C₁₋₁₀-alkyl, phenyl or naphthyl, or represents optionally substituted alkylene having from 1 to 4 C atoms, or other bifunctional groups which permit A and Z, together with the atoms to which they are bonded, to form a saturated or unsaturated heterocyclic ring,
E represents NO₂, CN or halogenoalkylcarbonyl such as 1,5-halogen-C₁₋₄-carbonyl;
X represents the -CH= or -N= radicals, with it being possible for the -CH= radical to be linked to the Z radical instead of an H atom;
Z represents groups which permit Z, together with the atom to which it is bonded and the =C- radical in place of X, to form a saturated or unsaturated heterocyclic ring,
for preparing agents for controlling fish-parasitizing lice in fish.

3. Use of compounds of the general formula (I) according to Claim 1, in which
R represents hydrogen or optionally substituted formyl, alkylcarbonyl, arylcarbonyl, alkylsulphonyl, arylsulphonyl, (alkyl-)-(aryl-)-phosphoryl, methyl, ethyl, i-propyl, sec- or t-butyl, phenyl, thiophenyl, furyl, thiazolyl, imidazolyl, pyridyl or benzthiazolyl or heteroarylmethyl, heteroarylethyl whose heteroaryl radical is thiophenyl, furyl, thiazolyl, imidazolyl or pyridyl;
A represents a monofunctional group from the series consisting of hydrogen and optionally substituted formyl, alkylcarbonyl, arylcarbonyl, alkylsulphonyl, arylsulphonyl, (alkyl-)-(aryl-)-phosphoryl, methyl, ethyl, i-propyl, sec- or t-butyl or phenyl, or represents optionally substituted alkylene having from 1 to 2 C atoms, or other bifunctional groups which permit A and Z, together with the atoms to which they are bonded, to form a saturated 5- to 7-membered heterocyclic ring which contains a further 1 or 2 identical or different heteroatoms and/or heterogroups,
E represents NO₂, CN or COCF₃;
X represents the -CH= or -N= radicals, with it being possible for the -CH= radical to be linked to the Z radical instead of an H atom;
Z represents groups which permit Z, together with the atom to which it is bonded and the =C- radical in place of X, to form a saturated or unsaturated 5- to 7-membered heterocyclic ring which contains a further 1 or 2 identical or different heteroatoms and/or hetero groups,
for preparing agents for controlling fish-parasitizing lice in fish.

4. Use of compounds of the general formula (I) according to Claim 1, in which
R represents hydrogen or optionally substituted formyl, alkylcarbonyl, arylcarbonyl, alkylsulphonyl, arylsulphonyl, (alkyl-)-(aryl-)-phosphoryl, methyl, ethyl, i-propyl, sec- or t-butyl, phenyl, thiophenyl, furyl, thiazolyl, imidazolyl, pyridyl or benzthiazolyl or heteroarylmethyl, heteroarylethyl whose heteroaryl radical is thiophenyl, furyl, thiazolyl, imidazolyl or pyridyl;
A represents a monofunctional group from the series consisting of hydrogen and optionally substituted formyl, alkylcarbonyl, arylcarbonyl, alkylsulphonyl, arylsulphonyl, (alkyl-)-(aryl-)-phosphoryl, methyl, ethyl, i-propyl, sec- or t-butyl or phenyl, or represents optionally substituted alkylene having from 1 to 2 C atoms, or other bifunctional groups which permit A and Z, together with the atoms to which they are bonded, to form a saturated or unsaturated 5- to 7-membered heterocyclic ring which contains a further 1 or 2 identical or different heteroatoms and/or heterogroups from the group consisting of oxygen, sulphur and nitrogen, with N-alkyl being the hetero group,
E represents NO₂, CN or COCF₃;
X represents the -CH= or -N= radicals, with it being possible for the -CH= radical to be linked to the Z radical instead of an H atom;
Z represents groups which permit Z, together with the atom to which it is bonded and the =C- radical in place of X, to form a saturated or unsaturated 5- to 7-membered heterocyclic ring which contains a further 1 or 2 identical or different heteroatoms and/or heterogroups from the group consisting of oxygen, sulphur and nitrogen, with N-alkyl being the hetero group,
for preparing agents for controlling fish-parasitizing lice in fish.

5. Use according to Claim 1 of compounds of the general formulae II and III: 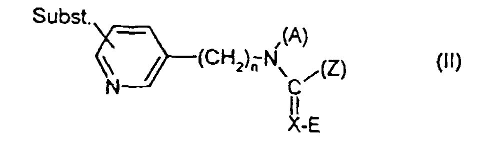 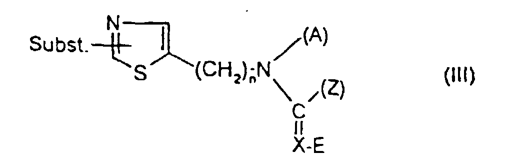 in which
n represents 1 or 2,
Subst. represents halogen,
A, Z, X and E have the meanings given in Claim 1,
for preparing agents for controlling fish-parasitizing lice in fish.

6. Use according to Claim 1 of imidaclopride of the formula 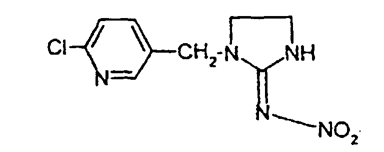 for preparing agents for controlling fish-parasitizing lice in fish.

7. Use of compounds according to Claims 1-6 for preparing agents for controlling fish-parasitizing lice from the genera Caligus, Lepeophtheirus and Argulus.

8. Use of compounds according to Claims 1-6 for preparing agents for controlling fish-parasitizing lice in Salmonidae.

## Revendications

1. Utilisation de composés de formule générale I 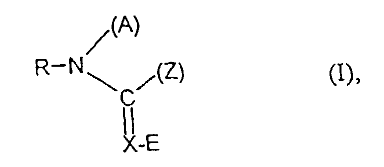 dans laquelle
R représente l'hydrogène, des restes acyle, alkyle, aryle, aralkyle, hétéroaryle ou hétéroarylalkyle éventuellement substitués ;
A représente un groupe monofonctionnel de la série hydrogène et restes acyle, alkyle ou aryle éventuellement substitués ou représente un groupe bifonctionnel qui est lié au reste Z ;
E est un reste électroattractif ;
X représente les restes -CH= ou -N=, le reste -CH= pouvant être lié à la place d'un atome d'hydrogène au reste Z ;
Z représente un groupe monofonctionnel de la série alkyle, -O-R, -S-R, -NR-R ou représente un groupe bifonctionnel qui est lié au reste A ou au reste X
pour la préparation de compositions destinées à combattre chez les poissons des crustacés qui les parasitent.

2. Utilisation de composés de formule générale (I) suivant la revendication 1, dans laquelle
R représente l'hydrogène, un groupe, éventuellement substitué, formyle, alkylcarbonyle, arylcarbonyle, alkylsulfonyle, arylsulfonyle, (alkyl-)-(aryl-)-phosphoryle, alkyle en C₁ à C₁₀, phényle, naphtyle, phénylméthyle, phénéthyle, hétéroaryle ayant jusqu'à 10 atomes de carbone dans le noyau et des atomes N, O, S, notamment N, comme hétéroatomes, ou hétéroarylméthyle, hétéroaryléthyle ayant jusqu'à 6 atomes dans le noyau et N, O, S, notamment N, comme hétéroatomes ;
A représente un groupe monofonctionnel de la série hydrogène et formyle, alkylcarbonyle, arylcarbonyle, alkylsulfonyle, arylsulfonyle, (alkyl-)-(aryl-)-phosphoryle, alkyle en C₁ à C₁₀, phényle, naphtyle, ou un groupe alkylène, éventuellement substitué, ayant 1 à 4 atomes de carbone ou d'autres groupes bifonctionnels qui permettent à A et Z, conjointement avec les atomes auxquels ils sont liés, de former un noyau hétérocyclique saturé ou non saturé,
E représente NO₂, CN, un groupe halogénalkylcarbonyle tel que 1,5-halogéno-carbonyle en C₁ à C₄ ;
X représente les restes -CH= ou -N=, le reste -CH= pouvant être lié au reste Z à la place d'un atome d'hydrogène ;
Z représente des groupes qui permettent à Z de former un noyau hétérocyclique saturé ou non saturé conjointement avec l'atome auquel il est lié et avec le reste =C- à la place de X,
pour la préparation de compositions destinées à combattre chez des poissons des crustacés qui les parasitent.

3. Utilisation de composés de formule générale (I) suivant la revendication 1, dans laquelle
R représente l'hydrogène, un groupe, éventuellement substitué, formyle, alkylcarbonyle, arylcarbonyle, alkylsulfonyle, arylsulfonyle, (alkyl-)-(aryl-)-phosphoryle, méthyle, éthyle, isopropyle, sec.-butyle, tertio-butyle, phényle, thiophényle, furyle thiazolyle, imidazolyle, pyridyle, benzothiazolyle ou hétéroarylméthyle, hétéroaryléthyle, avec comme reste hétéroaryle un reste thiophényle, furyle, thiazolyle, imidazolyle, pyridyle ;
A représente un groupe monofonctionnel de la série hydrogène et formyle, alkylcarbonyle, arylcarbonyle, alkylsulfonyle, arylsulfonyle, (alkyl-)-(aryl-)-phosphoryle, méthyle, éthyle, isopropyle, sec.-butyle, tertio-butyle, phényle éventuellement substitué ou un reste alkylène en C₁ ou C₂ éventuellement substitué, ou d'autres groupes bifonctionnels qui permettent à A et Z de former avec les atomes auxquels ils sont liés, un noyau hétérocyclique saturé pentagonal à heptagonal qui contient 1 ou 2 autres hétéroatomes et/ou hétérogroupes identiques ou différents,
E représente NO₂, CN ou COCF₃ ;
X représente les restes-CH= ou -N=, le reste -CH= pouvant être lié au reste Z à la place d'un atome d'hydrogène ;
Z représente des groupes qui permettent à Z de former conjointement avec l'atome auquel il est lié et avec le reste =C- à la place de X un noyau hétérocyclique pentagonal à heptagonal saturé ou non saturé qui contient 1 ou 2 autres hétéroatomes et/ou hétérogroupes identiques ou différents,
pour la préparation de compositions destinées à combattre chez des poissons des crustacés qui les parasitent.

4. Utilisation de composés de formule générale (I) suivant la revendication 1, dans laquelle
R représente l'hydrogène, un groupe, éventuellement substitué, formyle, alkylcarbonyle, arylcarbonyle, alkylsulfonyle, arylsulfonyle, (alkyl-)-(aryl-)-phosphoryle, méthyle, éthyle, isopropyle, sec.-butyle, tertio-butyle, phényle, thiophényle, furyle thiazolyle, imidazolyle, pyridyle, benzothiazolyle ou hétéroarylméthyle, hétéroaryléthyle, avec comme reste hétéroaryle un reste thiophényle, furyle, thiazolyle, imidazolyle, pyridyle ;
A représente un groupe monofonctionnel de la série hydrogène et formyle, alkylcarbonyle, arylcarbonyle, alkylsulfonyle, arylsulfonyle, (alkyl-)-(aryl-)-phosphoryle, méthyle, éthyle, isopropyle, sec.-butyle, tertio-butyle, phényle éventuellement substitué ou un reste alkylène en C₁ ou C₂ éventuellement substitué, ou d'autres groupes bifonctionnels qui permettent à A et Z de former conjointement avec les atomes auxquels ils sont liés un noyau hétérocyclique pentagonal à heptagonal saturé ou non saturé qui contient 1 ou 2 autres hétéroatomes et/ou hétérogroupes, identiques ou différents, de la série oxygène, soufre ou azote et, comme hétérogroupes, des groupes N-alkyle,
E représente NO₂, CN ou COCF₃ ;
X représente les restes -CH= ou -N=, le reste -CH= pouvant être lié au reste Z à la place d'un atome d'hydrogène ;
Z représente des groupes qui permettent à Z de former conjointement avec l'atome auquel il est lié et avec le reste =C- à la place de X un noyau hétérocyclique pentagonal à heptagonal saturé ou non saturé qui contient 1 ou 2 autres hétéroatomes et/ou hétérogroupes identiques ou différents, de la série oxygène, soufre ou azote et comme hétérogroupe un groupe N-alkyle,
pour la préparation de compositions destinées à combattre chez des poissons des crustacés qui les parasitent.

5. Utilisation suivant la revendication 1 de composés de formules générales II et III : 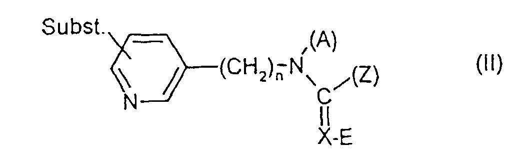 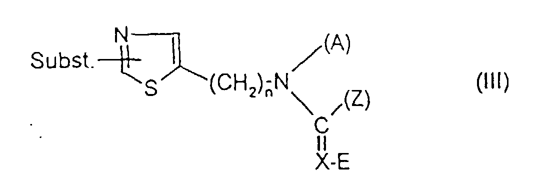 dans lesquelles
n a la valeur 1 ou 2,
Subst. représente un halogène,
A, Z, X et E ont les définitions indiquées dans la revendication 1,
pour la préparation de compositions destinées à combattre chez des poissons des crustacés qui les parasitent.

6. Utilisation suivant la revendication 1 de l'Imidacloprid de formule 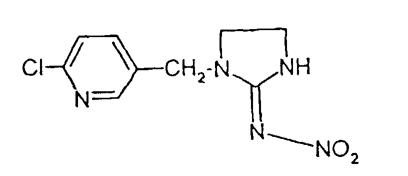 pour la préparation de compositions destinées à combattre chez des poissons des crustacés qui les parasitent.

7. Utilisation de composés suivant les revendications 1 à 6 pour la préparation de compositions destinées à combattre des crustacés des genres Caligus, Lepeophtheirus et Argulus parasitant les poissons.

8. Utilisation de composés suivant les revendications 1 à 6 pour la préparation de compositions destinées à combattre des crustacés parasitant des poissons de la famille des Salmonidae.
